# EUROPEAN PATENT SPECIFICATION

(11) **EP 0 871 664 B1**
(45) Date of publication and mention of the grant of the patent: **02.01.2004**
(21) Application number: 96936873.7
(22) Date of filing: 23.10.1996
(51) Int. Cl.: C07K 14/52, A61K 38/19

(54) **MOBILIZATION OF HEMATOPOIETIC STEM CELLS USING A CHEMOKINE**
MOBILISIERUNG VON HAEMATOPOIETISCHEN STAMMZELLEN DURCH EIN CHEMOKIN
MOBILISATION DE CELLULES SOUCHES HEMATOPOIETIQUES PAR UN CHIMIOKINE

(30) Priority: 24.10.1995 US 6051 P
(43) Date of publication of application: 21.10.1998
(60) Divisional application: 03022784.7
(73) Proprietor: SMITHKLINE BEECHAM CORPORATION, King of Prussia, PA 19406-0939 (US); Human Genome Sciences, Inc., Rockville, MD 20850 (US)
(72) Inventor: WHITE, John, R., Coatesville, PA 19320 (US); PELUS, Louis, Richboro, PA 18954 (US); LI, Haodong, Gaithersburg, MD 20878 (US); KREIDER, Brent, L., Germantown, MD 20874 (US)
(74) Representative: Crump, Julian Richard John
(86) International application number: PCT/US1996/016959
(87) International publication number: WO 1997/015594

(56) References cited:
- WO-A-95/17092
- WO-A-95/18228
- BLOOD, 01 March 1992, Vol. 79, No. 5, BRUGGER et al., "Mobilization of Peripheral Blood Progenitor Cells by Sequential Administration of Interleukin-3 and Granulocyte-macrophage Colony-stimulating Factor Following Polychemotherapy With Etoposide, Ifosfamide and Cisplatin", pages 1193-1200.
- TIPS, May 1994, Vol. 15, HORUK R., "Molecular Properties of the Chemokine Receptor Family", pages 159-165.
- BLOOD, 15 April 1995, Vol. 85, No. 8, LATERVEER et al., "Interleukin-8 Induces Rapid Mobilization of Hematopoietic Stem Cells With Radioprotective Capacity and Longterm Myelolymhoid Repopulating Ability", pages 2269-2275.

### SEQUENCE LISTING

(1) GENERAL INFORMATION:
   (i) APPLICANT: White et al.
   (ii) TITLE OF INVENTION: Novel Chemokine for Mobilizing Stem Cells
   (iii) NUMBER OF SEQUENCES: 6
   (iv) CORRESPONDENCE ADDRESS:
      (A) ADDRESSEE: SmithKline Beecham Corporation
      (B) STREET: 709 Swedeland Road, P.O. Box 1539
      (C) CITY: King of Prussia
      (D) STATE: PA
      (E) COUNTRY: USA
      (F) ZIP: 19406-0939
   (v) COMPUTER READABLE FORM:
      (A) MEDIUM TYPE: DISKETTE, 3.5 INCH, 1.44 Mb STORAGE
      (B) COMPUTER: IBM 486
      (C) OPERATING SYSTEM: WINDOWS FOR WORKGROUPS
      (D) SOFTWARE: WORD PERFECT 5.1
   (vi) CURRENT APPLICATION DATA:
      (A) APPLICATION NUMBER: not yet assigned
      (B) FILING DATE: Herewith
      (C) CLASSIFICATION:
   (vii) PRIOR APPLICATION DATA:
      (A) APPLICATION NUMBER: 60/006.051
      (B) FILING DATE: October 24, 1995
   (viii) ATTORNEY/AGENT INFORMATION:
      (A) NAME: William T. Han
      (B) REGISTRATION NUMBER: 34,344
      (C) REFERENCE/DOCKET NUMBER: P 50382
   (ix) TELECOMMUNICATION INFORMATION:
      (A) TELEPHONE: 610-270-5024
      (B) TELEFAX: 610-270-5090
(2) INFORMATION FOR SEQ ID NO: 1:
   (i) SEQUENCE CHARACTERISTICS:
      (A) LENGTH: 72
      (B) TYPE: Amino Acid
      (D) TOPOLOGY: Linear
   (xi) SEQUENCE DESCRIPTION: SEQ ID NO: 1:
   THR LYS THR GLU SER SER SER ARG GLY PRO TYR HIS PRO SER GLU
   1               5                   10                  15
   CYS CYS PHE THR TYR THR THR TYR LYS ILE PRO ARG GLN ARG ILE
                   20                  25                  30
   MET ASP TYR TYR GLU THR ASN SER GLN CYS SER LYS PRO GLY ILE
                   35                  40                  45
   VAL PHE ILE THR XAA ARG GLY HIS SER VAL CYS THR ASN PRO SER
                   50                  55                  60
   ASP LYS TRP VAL GLN ASP TYR ILE LYS ASP MET LYS
                   65                  70
(2) INFORMATION FOR SEQ ID NO: 2:
   (i) SEQUENCE CHARACTERISTICS:
      (A) LENGTH: 69
      (B) TYPE: Amino Acid
      (D) TOPOLOGY: Linear
   (xi) SEQUENCE DESCRIPTION: SEQ ID NO: 2:
   ALA SER PRO TYR SER SER ASP THR THR PRO CYS CYS PHE ALA TYR
   1               5                   10                  15
   ILE ALA ARG PRO LEU PRO ARG ALA HIS ILE LYS GLU TYR PHE TYR
                   20                  25                  30
   THR SER GLY LYS CYS SER ASN PRO ALA VAL VAL PHE VAL THR ARG
                   35                  40                  45
   LYS ASN ARG GLN VAL CYS ALA ASN PRO GLU LYS LYS TRP VAL ARG
                   50                  55                  60
   GLU TYR ILE ASN SER LEU GLU MET SER
                   65
(2) INFORMATION FOR SEQ ID NO: 3:
   (i) SEQUENCE CHARACTERISTICS:
      (A) LENGTH: 69
      (B) TYPE: Amino Acid
      (D) TOPOLOGY: Linear
   (xi) SEQUENCE DESCRIPTION: SEQ ID NO: 3:
   ALA PRO TYR GLY ALA ASP THR PRO THR ALA CYS CYS PHE SER TYR
   1               5                   10                  15
   SER ARG LYS ILE PRO ARG GLN PHE ILE VAL ASP TYR PHE GLU THR
                   20                  25                  30
   SER SER LEU CYS SER GLN PRO GLY VAL ILE PHE LEU THR LYS ARG
                   35                  40                  45
   ASN ARG GLN ILE CYS ALA ASP SER LYS GLU THR TRP VAL GLN GLU
                   50                  55                  60
   TYR ILE THR ASP LEU GLU LEU ASN ALA
(2) INFORMATION FOR SEQ ID NO: 4:
   (i) SEQUENCE CHARACTERISTICS:
      (A) LENGTH: 69
      (B) TYPE: Amino Acid
      (D) TOPOLOGY: Linear
   (xi) SEQUENCE DESCRIPTION: SEQ ID NO: 4:
   ALA PRO MET GLY SER ASP PRO PRO THR SER CYS CYS PHE SER TYR
   1               5                   10                  15
   THR SER ARG GLN LEU HIS ARG SER PHE VAL MET ASP TYR TYR GLU
                   20                  25                  30
   THR SER SER LEU CYS SER LYS PRO ALA VAL VAL PHE LEU THR LYS
                   35                  40                  45
   ARG GLY ARG GLN ILE CYS ALA ASN PRO SER GLU PRO TRP VAL THR
                   50                  55                  60
   GLU TYR MET SER ASP LEU GLU LEU ASN
                   65
(2) INFORMATION FOR SEQ ID NO: 5:
   (i) SEQUENCE CHARACTERISTICS:
      (A) LENGTH: 78
      (B) TYPE: Amino Acid
      (D) TOPOLOGY: Linear
   (xi) SEQUENCE DESCRIPTION: SEQ ID NO: 5:
   LEU ALA GLN PRO ASP ALA ILE ASN ALA PRO VAL THR CYS CYS TYR
   1               5                   10                  15
   ASN PHE THR ASN ARG LYS ILE SER VAL GLN ARG LEU ALA SER TYR
                   20                  25                  30
   ARG ARG ILE THR SER SER LYS CYS PRO LYS GLU ALA VAL ILE PHE
                   35                  40                  45
   LYS THR ILE VAL ALA LYS GLU ILE CYS ALA ASP PRO LYS GLN LYS
                   50                  55                  60
   TRP VAL GLN ASP SER MET ASP HIS LEU ASP LYS GLN THR GLN THR
                   65                  70                  75
   PRO LYS THR
(2) INFORMATION FOR SEQ ID NO: 6:
   (i) SEQUENCE CHARACTERISTICS:
      (A) LENGTH: 82
      (B) TYPE: Amino Acid
      (D) TOPOLOGY: Linear
   (xi) SEQUENCE DESCRIPTION: SEQ ID NO: 6:
   SER PRO GLN GLY LEU ALA GLN PRO VAL GLY ILE ASN THR SER THR
   1               5                   10                  15
   THR CYS CYS TYR ARG PHE ILE ASN LYS LYS ILE PRO LYS GLN ARG
                   20                  25                  30
   LEU GLU SER TYR ARG ARG THR THR SER SER HIS CYS PRO ARG GLU
                   35                  40                  45
   ALA VAL ILE PHE LYS THR LYS LEU ASP LYS GLU ILE CYS ALA ASP
                   50                  55                  60
   PRO THR GLN LYS TRP VAL GLN ASP PHE MET LYS HIS LEU ASP LYS
                   65                  70                  75
   LYS THR GLN THR PRO LYS LEU
                   80

## Description

Hematopoietic cells have very important roles in a number of different processes in the body. For example, leukocytic hematopoietic cells are important in maintaining the body's defenses against disease; monocytes, macrophages and lymphocytes are involved in potentiating the body's responses to infection and tumors, while granulocytes are involved in overcoming infection, parasites and tumors. Platelets, another hematopoietic cell, form an important element in the hemostatic mechanism through initiating thrombus formation by their adhesion to each other and to damaged surfaces, and by the release of factors which assist in the formation of the fibrin clot. Erythrocytes are mainly involved in the transport of oxygen.

All of these blood cells are derived from a single progenitor cell called the hematopoietic stem cell. Stem cells are both pluripotent, in that they give rise to all different cell types, and capable of self renewal. Hematopoietic stem cells make up only a small percentage of bone marrow cells and are normally quiescent. However, when stimulated to divide, these stem cells produce a differentiated daughter cell with great proliferative potential. Sequential rounds of division and differentiation give rise to an enormous amplification of cell numbers which is necessary for the production of mature blood cells. This process of division and differentiation is subject to regulation at many levels to control cell production.

Numerous studies have led to the definition of functions of several hematopoietic regulatory messengers. These biomolecules have been characterized as stimulatory, e.g., Colony Stimulating Factors (CSFs) and interleukins (IL-1, IL-3, IL-5 and IL-9); inhibitory, e.g., transforming growth factor-β (TGF-β), interferon, prostaglandin E, tumor necrosis factor, macrophage inflammatory protein-1 (MIP-1), lactoferrin, acidic isoferritins, AcSKDP, and pEEDCK (a synthetic HP5B monomer); or enhancing, e.g., TGF-β, IL-6, IL-4, IL-9, IL-11, MIP-1, MIP-2, leukemia inhibitory factor and *Steel* factor. Pelus *et al. Experimental Hematology* **1994, 22**:239-247. Stimulatory biomolecules have been found to promote division of particular cell lineages. For example, G-CSF derives neutrophil production, while erythropoietin promotes formation of erythrocytes.

A number of these biomolecules and additional agents have been found to induce the mobilization of hematopoietic stem cells.

A single injection of IL-8 has been shown to induce mobilization of pluripotent stem cells that are able to provide permanent reconstitution of myeloid cells and of T and B lymphocytes. Laterveer *et al. Blood* **1995, 85**(8):2269-2275. IL-8 belongs to a family of pro-inflammatory molecules called chemokines. This family has been divided into two subfamilies, the CXC and CC chemokines, based on whether the first two cysteine residues in a conserved motif are adjacent to each other or are separated by an intervening residue. In general, CXC, which include IL-8, melanoma growth-stimulating activity (MGSA) and platelet factor 4 (PF4), are potent chemoattractants and activators of neutrophils but not monocytes. In contrast, CC chemokines, which include RANTES, monocyte chemotactic protein 1 (MCP-1) and MIP-1, are chemoattractants for monocytes but not neutrophils.

Stem cell inhibitors (SCIs) such as the CC chemokines, murine and human MIP-1α (LD78), have also been shown to enhance the release and mobilization of cells into the peripheral blood. WO 94/28916; Simm *et al. Blood* **1994, 84**:2937.

Increased mobilization of stem cells in patients treated with sequentially administered interleukin-3 and GM-CSF compared with GM-CSF alone has been reported by Brugger *et al. Blood* **1992, 79**:1193-1200. In addition, it has been shown that the absolute number of peripheral blood progenitor cells can be expanded *in vitro* by culture in a cocktail of cytokines, usually including SCF, IL-3, and either IL-6 or IL-1. Bodine, D. *Experimental Hematology* **1995, 23**:293-295.

SK&F 107647, a hematoregulatory agent containing an ethylene bridge in place of the cysteine bridge of HP5B, has been demonstrated to be a potent stimulator of *in vitro* myelopoiesis. Pelus *et al. Experimental Hematology* **1994, 22**:239-247. Injection of SK&F 107647 in normal mice resulted in a two- to six-fold increase in serum colony-stimulating activity. Administration of this agent over 4 days resulted in significant increases in the number of granulocyte-macrophage, erythroid, and multipotential progenitor cells, as well as stimulating their cell cycle rates.

It has also been found that pretreatment with stem cell stimulating factor such as G-CSF can expand the pool of progenitor cells susceptible for mobilization by these agents, further increasing their mobilizing effect. For example, the combination of MIP-1α with G-CSF was found to increase white cell count in the blood as compared to G-CSF alone. Simm *et al. Blood* **1994, 84**:2937. Co-administration of SCI with G-CSF caused the enhanced mobilization of a number of cell types including neutrophils, monocytes, eosinphils, lymphocytes and basophils. WO 94/28916. Administration of G-CSF alone had no effect on the release of eosinphils or basophils after 2 days of administration. Similar effects were observed when other agents such as GM-CSF, f-MET-Leu-Phe or IL-8 were coadministered with SCIs.

In recent years, the availability of recombinant cytokines and the use of hematopoietic stem cell support have resulted in the widespread application of high-dose chemotherapy regimens designed to improve the success of cancer therapy. Despite significant advances, however, delayed recovery of hematopoiesis remains an important source of morbidity and mortality for patients treated with this approach. Since their discovery over 20 years ago, peripheral blood hematopoietic progenitor cells (PBPCs) have been increasingly used to supplement and even replace bone marrow as the source of hematopoietic support in a variety of situations.

Purified populations of cells are increasingly being used therapeutically and it would therefore be advantageous to be able to increase the number of circulating blood cells. It is useful to be able to harvest hematopoietic cells prior to chemotherapy or radiotherapy, thus, protecting them from harmful effects of this therapy; after therapy, the cells can be returned to the patient. It would therefore be highly beneficial to provide an agent which promoted the release and mobilization of a number of hematopoietic cells. Such an agent would be useful for enhancing the response to infection.

According to the present invention there is provided the use of an effective amount of a polypeptide comprising SEQ ID NO:1 in the manufacture of a medicament for administration to an animal in need thereof for mobilizing hematopoietic stem cells.

Peripheral blood cell transplantation is an important procedure in the treatment of cancer patients with high dose chemotherapy. In such treatment, patients are treated to induce clinical remission of their cancer, then during the remission, successive treatment with CSF, for example, by priming with cyclophosphamide then administration of G-CSF, causes eventual mobilization of cells from the bone marrow to the peripheral circulation for harvesting of leukophoresed blood; then the patient is given high dose chemotherapy or radiotherapy and the resultant bone marrow failure is compensated for by infusion of the stored blood or cells collected previously. This procedure may be modified by the omission of the initial induction of remission, and whole blood may be collected rather than leukophoresed blood. The mobilization effects of the present invention makes it a candidate both to replace CSFs in such cancer treatment regimes, and also to complement the mobilization effects of CSFs in combined treatments.

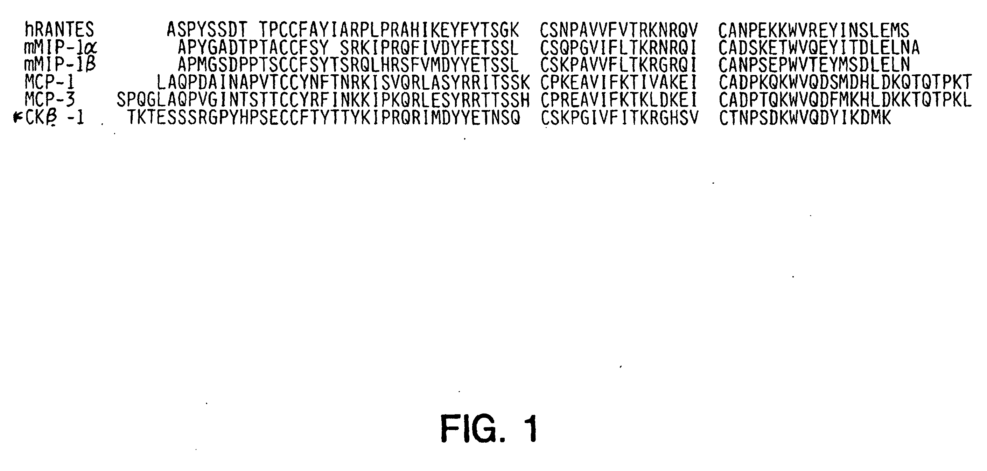

The two subfamilies of chemokines (CXC and CC) are ever expanding and presumably the individual members have similar, if slightly divergent, functions. A chemokine which may be used in accordance with the present invention (Ckβ-1) is a member of the CC subfamily and is structurally similar to MCP-1, MCP-3, hRANTES, mMIP-1α, and mMIP-1β. Figure 1 shows the sequence and alignment of Ckβ-1 with other known chemokines. WO 95/18228 A1 discloses a CC-1 cytokine which is similar to Ckβ-1, having two additional amino acid residues at the C-terminus. WO 95/17092 A1 discloses another analogue of Ckβ-1, called MIP-1γ, having an additional nineteen N-terminus amino acids and two fewer amino acids at the C-terminus.

The effect of a polypeptide comprising SEQ ID NO:1 in inducing Ieukophilia will find clinical and veterinary application in all utilities where the raising of hematopoietic cell levels is important. For example, said polypeptide can be used to enhance immune responses against chronic infections, particularly parasitic and bacterial infections. It may also have a role in promoting wound healing.

The chemoattractant activity of a polypeptide comprising SEQ ID NO:1 can be boosted by pretreatment with a colony stimulating factor such as G-CSF or GM-CSF. Alternatively, the hematoregulatory peptides SK&F 107647 (currently in clinical trials), FLT-3 ligand (Immunex) or any other G-CSF mimetics (peptide and non-peptide) may be used. These stimulants may have an even more dramatic effect on said polypeptide than on those already known due to their slight structural differences. As known in the art, these peptides are useful in stimulating myelopoiesis in patients suffering from reduced myelopoietic activity, including bone marrow damage, agranulocytosis and aplastic anemia. Also included are patients who have depressed bone marrow function due to immunosuppressive treatment to suppress tissue reactions (i.e., bone marrow transplant surgery). They may also be used to promote more rapid regeneration of bone marrow after cytostatic chemotherapy and radiation therapy for neoplastic and viral diseases. There may also be a value where patients have serious infections due to a lack of immune response following bone marrow failure.

The hematopoietic stem cells released and harvested in the manner described above may be useful for subsequent *in vitro* and *ex vivo* manipulations to deliver gene products in gene therapy. Another embodiment is co-administration with cytotoxic drugs.

The following examples are provided for illustrative purposes only and are not intended to limit the invention.

### Drawings

Figure 1 shows the sequence of cytokine CKβ-1 alligned to several known chemokines

### EXAMPLES

### Example 1: Mobilization Assay for Novel Chemokines as Single Agents

Ckβ-1 will be tested as an individual stem cell mobilization agent in BDF 1 mice. Ckβ-1 will be assayed in concentrations of 50, 10, and 2 µg/mouse and administered via SC, IM, or a PO route. The kinetics of chemokine mobilization of stem cells will be monitored in 15 minute intervals over a period of 60 minutes by collecting blood samples from the mice by cardiac puncture. The mobilized stem cells will be collected by a densing gradient (Lympholyte M). Cells are washed then frozen for future usage. The mobilization profile of the blood differentials will be assessed using a Technicon H1 hematology analyzer. Mobilization of inflammatory cells such as PMN's, eosinophils, and basophils will be taken into account when evaluating the overall potential inflammatory profile. The chemokine IL-8, which mobilizes hematopoietic stem cells as a single factor, will be included in these studies as a positive control.

### Example 2: Mobilization Assay for Novel Chemokines in Combination with Hematostimulants

In these studies, hematostimulants will be assayed in combination with Ckβ-1 as mobilization factors. These agents include: G-CSF, GM-CSF, SK&F 107647, and FLT-3 ligand. However, any G-CSF mimetic (hematostimulants which are not colony stimulating factors like G-CSF or GMCSF, but have hematopoietic activity) may be used. In combination studies, G-CSF will be administered IP to mice four days prior to Ckβ-1. As in Example 1, the dose of chemokine and time of blood collection will be varied. Combination studies with hematostimulant pre-treatment will utilize MIP-1α as the positive control.

### Example 3: CFU Assay

Blood samples collected during the mobilization phase will be assessed for colony forming units (CFU-GM) at days 7 and 14. Cells are adjusted to 2X10⁶ cells/ml in McCoys medium with 15 x FBS serum. A single layer agar system utilizing the following is used: McCoys medium enriched with nutrients (NaHCO₃, pyruvate, amino acids and vitamins); 0.3% Bacto agar. To this is added cells from the blood samples (final concentration = 2X10⁵ cells/ml). The agar plates are incubated at 37°C, 5% CO₂ for 7 days. Colonies of proliferating cells (CFU-GM) are counted utilizing a microscope. In addition, early hematopoietic high proliferative potential (HPP) progenitors, will be counted in the day 14 CFU cultures.

## Claims

1. Use of an effective amount of a polypeptide comprising SEQ ID NO: 1 in the manufacture of a medicament for administration to an animal in need thereof for mobilizing hematopoietic stem cells.

2. The use of claim 1, wherein said medicament is adapted for administration with a cytotoxic drug.

3. The use of claim 1, wherein said medicament is adapted for administration with a colony stimulating factor.

4. The use of claim 1, wherein said medicament is adapted for administration with a hemoregulatory agent.

5. The use of any preceding claim, wherein said polypeptide consists of the polypeptide of SEQ ID NO: 1.

## Patentansprüche

1. Verwendung einer wirksamen Menge eines Polypeptids, das die Sequenz des Sequenzprotokolls SEQ ID N°:1 umfasst, bei der Herstellung eines Arzneimittels zur Verabreichung an behandlungsbedürftige Säuger einschließlich des Menschen zur Mobilisierung hämatopoetischer Stammzellen.

2. Verwendung nach Anspruch 1, wobei das Arzneimittel zur Verabreichung mit einem cytotoxischen Mittel geeignet ist.

3. Verwendung nach Anspruch 1, wobei das Arzneimittel zur Verabreichung mit einem Kolonie-stimulierenden Faktor geeignet ist.

4. Verwendung nach Anspruch 1, wobei das Arzneimittel zur Verabreichung mit einem hämoregulatorischen Mittel geeignet ist.

5. Verwendung nach einem oder mehreren der vorhergehenden Ansprüche, wobei das Polypeptid aus dem Polypeptid mit der Sequenz des Sequenzprotokolls SEQ ID N°:1 besteht.

## Revendications

1. Utilisation d'une quantité efficace d'un polypeptide comprenant la SEQ ID NO: 1 dans la fabrication d'un médicament pour l'administration à un animal qui en a besoin pour mobiliser les cellules souches hématopoïétiques.

2. Utilisation selon la revendication 1, dans laquelle ledit médicament est adapté à l'administration avec un produit cytotoxique.

3. Utilisation selon la revendication 1, dans laquelle ledit médicament est adapté à l'administration avec un facteur de croissance hématopoïétique.

4. Utilisation selon la revendication 1, dans laquelle ledit médicament est adapté à l'administration avec un agent hémorégulateur.

5. Utilisation selon l'une quelconque des revendications précédentes, dans laquelle ledit polypeptide consiste en le polypeptide de SEQ ID NO: 1.
